# EUROPEAN PATENT APPLICATION

(11) **EP 2 042 167 A1**
(43) Date of publication of application: **01.04.2009**
(21) Application number: 07291145.6
(22) Date of filing: 26.09.2007
(51) Int. Cl.: A61K 31/015, A61K 31/045, A61K 31/121, A61K 31/122, A61K 31/19, A61K 31/22, A61P 17/02, A61P 17/04, A61P 17/06

(54) **Use of a monoterpene to induce tissue repair**

(71) Applicant: AISA THERAPEUTICS, 91058 Evry Cedex (FR)
(72) Inventor: D'Alessio, Patrizia, 75007 Paris (FR)
(74) Representative: Colombet, Alain André

(57) **Abstract**

The invention relates to the use of a monoterpene to increase biological tissue repair, i.e. tissue regeneration or healing, the cosmetic use of a monoterpene to increase biological tissue repair of skin or a mucosa in an individual and the use of a monoterpene for the manufacture of a pharmaceutical composition intended to prevent or treat a biological tissue injury in an individual.

## Description

The invention relates to the use of a monoterpene to increase biological tissue repair.

Tissue repair includes the processes reaching to the recovery of a lesion, with or without relapse, by tissue regeneration or healing.

Intrinsic tissue regeneration is the body's regular maintenance in which millions of cells constantly produce tissue remodelling and restoration of tissue functions. It begins with stem cells. Biochemical signals draw the stem cells to sites where growth factors and cytokines have created an environment for regeneration.

Scar tissue is different from regenerated tissue. When an injury occurs, the body's first reaction is haemostasis when fibrin and inflammatory cytokines form a blood clot or provisional scaffold. More inflammatory cells arrive, remodelling the clot into scar tissue. Collagen in scar tissue is abnormally aligned and has little elastin. Unlike regenerated tissue, scar tissue is different-and less perfect- than the surrounding tissue it replaces.

Terpenes are a large and varied class of hydrocarbons, produced primarily by a wide variety of plants, particularly conifers, though also by some insects such as swallowtail butterflies, which emit terpenes from their osmeterium. They are the major components of resin, and of turpentine produced from resin. The name "terpene" is derived from the word "turpentine". When terpenes are modified chemically, such as by oxidation or rearrangement of the carbon skeleton, the resulting compounds are generally referred to as terpenoids. Some authors will use the term terpene to include all terpenoids. Terpenes and terpenoids are the primary constituents of the essential oils of many types of plants and flowers. Essential oils are used widely as natural flavor additives for food, as fragrances in perfumery, in aromatherapy, and in traditional and alternative medicines. Synthetic variations and derivatives of natural terpenes and terpenoids also greatly expand the variety of aromas used in perfumery and flavors used in food additives.

Terpenes are derived biosynthetically from units of isoprene, which has the molecular formula C₅H₈. The basic molecular formulas of terpenes are multiples of that, (C₅H₈)ₙ where n is the number of linked isoprene units. Isoprene itself does not undergo the building process, but rather activated forms, isopentenyl pyrophosphate (IPP or also isopentenyl diphosphate) and dimethylallyl pyrophosphate (DMAPP or also dimethylallyl diphosphate), are the components in the biosynthetic pathway. As chains of isoprene units are built up, the resulting terpenes are classified sequentially by size as hemiterpenes, monoterpenes, sesquiterpenes, diterpenes, sesterterpenes, triterpenes, and tetraterpenes.

Monoterpenes consist of two (repris du projet precedent) isoprene units and have the molecular formula C₁₀H₁₆. Examples of monoterpenes are: geraniol and limonene. Monoterpenes occur in monocyclic, bicyclic, and acyclic forms and are either simple or modified hydrocarbons. They are a class of isoprenoid molecules derived from the anabolism of acetate by the mevalonic acid branch biosynthetic pathways of plants. D-Limonene ((4R)-1-methyl-4-isopropenylcyclohex-1-ene), a major component of orange peel oil and the prototype of monoterpenes in carcinogenesis studies, is formed by the cyclization of the 10-carbon isoprene intermediate geranylpyrophosphate.

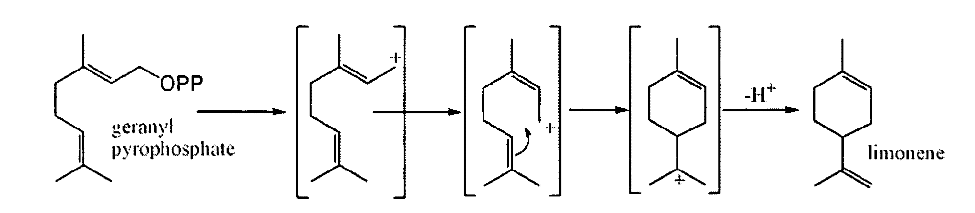

D-Limonene and its derived metabolites have been shown to possess cancer chemotherapeutic and chemopreventive efficacy in various preclinical model systems.

Crowell PL et al. identified plasma metabolites of limonene in blood of seven healthy human volunteers having ingested 100 mg/kg limonene in a custard. On-line capillary gas chromatography/mass spectrometry analysis indicated that at least five compounds were present at 4 h after ingestion. Two major peaks were identified as the rat limonene metabolites dihydroperillic acid and perillic acid, and two minor peaks were found to be the respective methyl esters of these acids (Crowell PL et al., 1994).

Although R- and S-limonene are only weak inhibitors of the isoprenylation enzymes, their major metabolites, perillic acid and perillyl alcohol, are more potent inhibitors, with IC50 values in the low mM range. The metabolites possess greater activity towards the geranylgeranyltransferase type I enzyme than farnesyltransferase (Hardcastle IR et al., 1999).

D-Limonene has a pronounced chemotherapeutic activity and minimal toxicity in preclinical studies. A phase I clinical trial to assess toxicity, the maximum tolerated dose (MTD) and pharmacokinetics in patients with advanced cancer was followed by a limited phase II evaluation in breast cancer. D-Limonene is well tolerated in cancer patients at doses which may have clinical activity (Vigushin DM et al., 1998).

Chemical compounds derived from plants used in traditional medicine to cure disease, are an important source for the development of new active pharmaceutical molecules. Using such a strategy, the inventors have previously identified several monoterpenes able to specifically prevent or treat the senescence of human vascular endothelial cells induced by repeated inflammatory episodes (WO 2005105074).

The inflammatory response is the first vitally necessary mechanism of tissue repair of injured tissues. It is characterized by immediate events to prevent loss of red blood cells, an acute response to remove damaged tissues and establish a vascular supply to support repair and a tissue repair phase.

Nonsteroidal anti-inflammatory drugs, like ibuprofen, an anti-inflammatory molecule on the market, impede the last stage of inflammation process, i.e. tissue repair, by virtue of retarding inflammation. Anti-inflammatory agents whose principal effect is to diminish granulocytic inflammatory reaction also have healing-depressant propensity (Lee KH et al. 1968).

Nonsteroidal anti-inflammatory drugs that decrease breaking strength in incision wounds were found to have variable effects on wound contraction, and epithelialization. (Rao CM. et al. 1988, Kumar A. et al. 1988).

Ibuprofen was reported to reduce the breaking strength of the repaired extensor tendon, wound contraction, and epithelialization (Dvivedi S. et al. 1997, Kulick MI. et al. 1986, Dong Y-L. et al. 1993).

The present inventors have now unexpectedly shown that limonene and perillyl alcohol induce biological tissue repair despite their anti-inflammatory effect previously observed by the present inventors (W02005/105074).

First, the present inventors have now unexpectedly observed in a rat colon inflammation model that the anti-inflammatory effect of limonene does not impede tissue repair.

Second, the present inventors have shown that limonene and perillyl alcohol are beneficial against chronic skin inflammation disorders like atopic dermatitis.

Third, the present inventors have shown the beneficial effect of limonene and perillyl alcohol in a classical model of healing.

In order to appreciate the effects of limonene on tissue repair, 30 Wistar HsdBrIHan female rats were used in a colitis rat model. Colon inflammation has been induced by a single rectal administration of an acid solution of sulfonic 2,5,6 - trinitrobenzene (TNBS, Fluka, France) and the anti-inflammatory effect of limonene on tissue repair given by oral administration was studied. Average macroscopic and microscopic scores of the colons sampled 6 days after the induction of colon inflammation were compared. The results have shown that limonene, compared with ibuprofen, induces post-inflammatory tissue repair, despite its anti-inflammatory effect. Moreover, it decreases the circulating TNF-α level at a concentration five times inferior to the concentrations of Ibuprofen, used as a reference molecule.

In a model of chronic skin inflammation, the inventors have also shown a significant effect of limonene and perillyl alcohol on skin repair and pro-inflammatory cytokines levels. Chronic skin inflammation has been induced in 24 female mice hairless Skh-1 by dorsal daily applications of Phorbol 12-myristate-13-acetate (TPA) during seven days (Stanley P.L. et al., 1991).

In a second skin model, the influence of limonene and perillyl alcohol on healing process was studied in hairless Skh-1 mice submitted to a scarification of the skin on each flank. Corn oil alone, limonene in solution in corn oil or perillyl alcohol in solution in corn oil were administered daily by topic application on each site of scarification at the dose of 10 mg/kg/day for 8 days. The wound healing process gave better results with daily applications of perillyl alcohol in comparison with limonene and much better results in comparison with the control.

### Use of a monoterpene to increase tissue repair

Thus, the present invention relates to the use of a monoterpene to increase biological tissue repair.

Preferably, tissue repair includes tissue regeneration and healing.

In one embodiment, a monoterpene is used according to the invention to increase biological tissue regeneration.

Preferably, a monoterpene is used according to the invention to increase biological tissue regeneration *in vitro.*

In the context of the present invention, a "tissue" means a collection of interconnected cells.

In the context of the present invention, a "biological tissue" means a tissue of human or animal origin and includes *in vivo* tissues and *in vitro* or *ex vivo* cultured tissues.

Examples of *in vitro* or *ex vivo* cultured tissues include cultured cells as fibroblasts and *in vitro* skin models which associate different cell types found in skin.

Examples of *in vivo* biological tissues include skin and mucosae.

In one embodiment, skin means scalp.

In the content of the present invention, a "biological tissue repair" means tissue regeneration and/or healing of a biological tissue showing injuries and/or defects and/or disease symptoms.

Biological tissue injuries include burns, cuts, burning relapses, wounds and relapses of extreme weather conditions including excessive cold or heat.

Biological tissue diseases include skin tissue diseases as atopic dermatitis, seborrhoic keratitis, epidermolysis bullosa acquisita, psoriasis, skin alterations in lupus erythematosus, dermatomyositis, scleroderma, chronic acne, chronic cellulites, pruritus and abnormal or defective scar formation as in diabetes.

Biological tissue defects include skin tissue defects such as acne pimples, cellulites, scars, sun and UV induced premature skin senescence, unesthetic skin defaults due to the aging process including scars and stains, wrinkles and squamous features, stretch marks, aging marks, sun and UV induced premature skin senescence, rosacea and unesthetisms of post-traumatic relapses.

The use of a monoterpene according to the invention to increase biological tissue repair may be cosmetic or therapeutic.

For such a use, the monoterpene may be administered by a systemic route or by a topic route. Preferably, the systemic route is selected from the group consisting of oral, lingual, sublingual, muscular and intravenous routes. More preferably, the systemic route is the oral route.

Preferably, a cosmetic use of a monoterpene according to the invention may be in the form of a cosmetic formulation used topically or in the form of a food complement used orally.

### Cosmetic use

In another embodiment, the invention relates to the cosmetic use of a monoterpene to increase biological tissue repair of skin or a mucosa in an individual.

Preferably, the invention relates to the use of a monoterpene orally or topically to increase biological tissue repair of skin or a mucosa in an individual.

Preferably, the invention relates to the cosmetic use of a monoterpene to increase tissue regeneration of skin or a mucosa in an individual.

Skin includes epidermis and dermis. Epidermis is the outermost layer of the skin. The main type of cells which make up the epidermis are keratinocytes, melanocytes, Langerhans cells and Merkels cells. The dermis is the layer of skin beneath the epidermis that consists of connective tissue.

Examples of mucosae include peri-oral mucosa, buccal mucosa, gastric and colon mucosa, genital mucosa.

As used herein, the term "individual" denotes a human, a pet, a laboratory animal or a farm animal, more preferably a bird or a mammal, such as a rodent, a feline, an equine, a bovine, a caprine, a canine, a porcine and a primate. Preferably, an individual according to the invention is a human, a cat or a dog.

"Topically" means applied to the skin or a mucosa.

In a further embodiment, the invention relates to a method for preventing or treating non-pathological conditions of skin or mucosa defects.

Preferably, a method for preventing or treating non-pathological conditions of skin or mucosa defects according to the invention comprises the application of a cosmetic formulation comprising a monoterpene, in one dose or in repeated doses and at specified intervals of time, on said skin or mucosa defect.

In the frame of methods for preventing or treating non-pathological conditions of skin or mucosa defects according to the invention, the administration regimen may be for instance for a period of more than 4 weeks, or more than 8 weeks.

Preferably, in the frame of methods for preventing or treating non-pathological conditions of skin or mucosa defects according to the invention, the range of dose of monoterpene may be between 0.1 mg/kg to 100 mg/kg/day. More preferably, the dose range is between 1 mg to 100 mg/kg. Most preferably, the dose range is between 10 mg/kg to 50 mg/kg.

Preferably, the cosmetic use of a monoterpene to increase biological tissue repair of skin or mucosa in an individual may be in a form selected in the group consisting of patch, capsule, pill, cream, paste (toothpaste), detergent such as shampoo and dermo-soap, bubble bath or bath salts, aqueous, alcoholic or oily lotion, gel, low ozone spray.

Preferably, the monoterpene is entrapped in liposomes or any other chemical or mechanical trapping system or device permitting a delayed effect.

The cosmetic use of a monoterpene according to the invention is preferably carried out topically by the application of a cosmetic formulation on skin or a mucosa.

Cosmetic formulations may include, besides a monoterpene, other active principles and one or more cosmetically acceptable carriers.

As used herein, «cosmetically acceptable carriers », means a carrier to be used in contact with the different superficial parts of the human body, for example epidermis, without toxicity, irritation, undue allergic response or other untoward reaction.

Such cosmetic formulations are generally in form of lotion, emulsion, cream, gel, shampoo or patch.

The one skilled in the art will be able to specify the supplementary components to be carried out in cosmetic formulations according to the type of cosmetic formulation to be obtained.

Preferably, the invention relates to the use of a monoterpene to increase skin or mucosa tissue repair on a skin or mucosa defect, said defect being preferably selected from the group consisting of unesthetic skin defaults such as acne pimples, cellulites, scars, sun and UV induced premature skin senescence, unesthetic skin defaults due to the aging process including scars and stains, wrinkles and squamous features, stretch marks, aging marks, sun and UV induced premature skin senescence, rosacea and unesthetisms of post-traumatic relapses.

### Pharmaceutical use

A further object of the present invention relates to the use of a monoterpene for the manufacture of a pharmaceutical composition intended to prevent or treat a biological tissue injury in an individual.

A further object of the present invention relates to the use of a monoterpene for the manufacture of a pharmaceutical composition intended to prevent or treat a biological tissue disease.

Preferably, said biological tissue injury or disease is a skin injury or disease.

In a preferred embodiment, said prevention or treatment concerns a human, a pet, a laboratory animal or a farm animal. More preferably, said prevention, alleviation or treatment concerns an individual selected in the group consisting of a bird or a mammal, such as a rodent, a feline, an equine, a bovine, a caprine, a canine, a porcine and a primate. Most preferably, said prevention, alleviation or treatment concerns a human, a cat or a dog.

Preferably, said pharmaceutical composition is used topically or systemically, more preferably orally.

Such pharmaceutical compositions comprise a monoterpene as active ingredient and pharmaceutically acceptable carriers.

"Pharmaceutically acceptable" refers to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the monoterpene used according to the invention, its use in the pharmaceutical compositions, in the medicaments, or for implementing the methods for preventing or treating a biological tissue injury in an individual according to the invention is contemplated. Supplementary active ingredients can also be incorporated into the pharmaceutical compositions.

In the context of the invention, the terms "to prevent", "preventing" or "prevention" means to allow avoiding a tissue injury.

In the context of the invention, the terms "to treat", "treating" or "treatment", means reversing, alleviating, or inhibiting a tissue injury.

In the context of the invention, a tissue disease is preferably a skin disease and may include skin tissue diseases as atopic dermatitis, seborrhoic keratitis, epidermolysis bullosa acquisita, psoriasis, skin alterations in lupus erythematosus, dermatomyositis, scleroderma, chronic acne, chronic cellulites, pruritus and abnormal or defective scar formation as in diabetes.

In the context of the invention, a tissue injury is preferably a skin injury and may include burns, cuts, relapses of extreme weather conditions including excessive cold or heat, burning relapses and wounds.

In a further embodiment, the invention relates to a method for preventing or treating a biological tissue injury or disease in an individual.

Preferably, a method for preventing or treating a biological tissue injury or disease in an individual according to the invention comprises the administration of a pharmaceutical composition comprising a monoterpene, in one dose or in repeated doses and at specified intervals of time.

In the frame of methods for preventing or treating a biological tissue injury or disease in an individual according to the invention, the administration regimen may be by a systemic or topic way, preferably by a topic way.

In the frame of methods for preventing or treating a biological tissue injury or disease in an individual according to the invention, the administration regimen may be for instance for a period of less than 6 weeks, or less than 8 weeks.

Preferably, in the frame of methods for preventing or treating a biological tissue injury or disease in an individual according to the invention, the range of dose of monoterpene may be between 0.1 mg/kg to 100 mg/kg/day. More preferably, the dose range is between 1 mg to 100 mg/kg. Most preferably, the dose range is between 10 mg to 50 mg/kg. More preferably, the dose range is between 10 mg/kg/day to 50 mg/kg/day.

The pharmaceutical use of a monoterpene according to the invention is preferably carried out topically by the application of a pharmaceutical formulation on skin or a mucosa.

Preferably, the use of a pharmaceutical composition comprising a monoterpene for the prevention or treatment of a biological tissue injury in an individual may be in a form selected in the group consisting of patch, capsule, pill, ointment and wound-dressing.

### Monoterpenes

Monoterpenes used in the present invention include limonene, geraniol, geranyl acetate, isomenthone, perillyl alcohol, perillic acid, dihydroperillic acid, the respective methyl esters of these acids, carvone, citral, menthol.

In the context of the present invention, limonene means (R)-(+)-limonene, i.e. D-limonene.

More preferably, monoterpenes used in the present invention are limonene, one of its metabolites or a mixture thereof.

Limonene metabolites include perillyl alcohol, perillic acid, dihydroperillic acid, and their respective methyl esters.

Most preferably, monoterpenes used in the present invention are limonene, perlillyl alcohol or perillic acid or a mixture thereof.

The invention will be further illustrated in view of the following figures and examples.

### FIGURES

Figure 1: Rats average weights evolution (Mean ± ESM)
Figure 2: Average serum levels of TNF-α (pg/ml) (Mean ± ESM) U Test Mann- Whitney : - *P* < 0.05 and ** P< 0.01 (vs. "Control") ; - # P < 0.05 (vs. "TNBS/Control") ;
Figure 3 : Average macroscopic scores of the rats colons of the 5 groups of treatment (Mean ± ESM) U Test Mann- Whitney : - *^{**}P* < 0.01 (vs. "Control") ; - ^{#}*P* < 0.05 and ^{##} *P* < 0.01 (vs. "TNBS/Control") ; - °*P* < 0.05 (*vs.* "TNBS/Limonene 100").
Figure 4 : Average colon lengths (cm) of the rats of the 5 groups of treatment (Mean ± ESM) U Test Mann- Whitney :-^{**}*P* < 0.01 (vs. "Control") ; - ^{#} P < 0.05 (vs. "TNBS/Control").
Figure 5 : Mice average weights evolution (Mean ± ESM)
Figure 6 : Average macroscopic scores of skin inflammation of the mice of the 4 groups of treatment
Figure 7 : Average microscopic scores of skin inflammation of the mice of the 4 groups of treatment
Figure 8 : Average serum levels of IL-1β (pg/ml) of the mice of the 4 groups of treatment before killing
Figure 9 : Average serum levels of IL-6 (pg/ml) of the mice of the 4 groups of treatment before killing

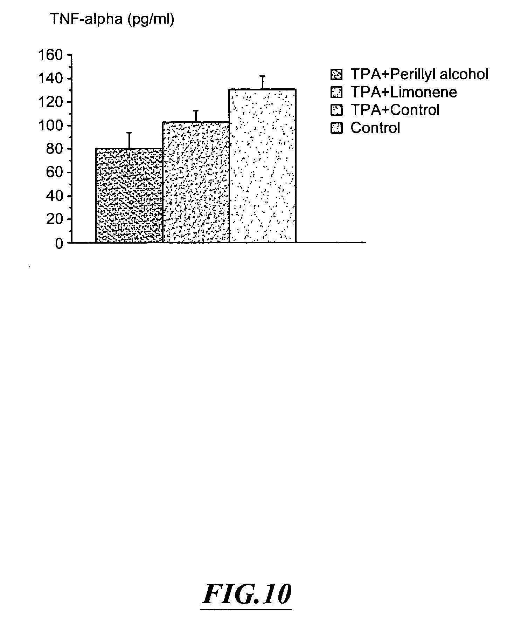

Figure 10 : Average serum levels of IFN-γ (pg/ml) of the mice of the 4 groups of treatment before killing

### Example 1 : preventive anti-inflammatory effects of limonene in a rat colitis model

### MATERIAL AND METHODS

### 1 - Animals

Thirty female rats Wistar HsdBrIHan EOPS (Ganat, France) with an average weight of 175-200 g are used.

### 2 - Assayed products

Limonene ((R)-(+)-Limonene, MW = 136.23, purity = 97%) and ibuprofen ((S)-(+)-Ibuprofen, MW = 206.28, purity = 100%) from SIGMA-ALDRICH (Saint-Quentin Fallavier, France) have been used. Limonene and Ibuprofen have been prepared in maize oil extemporarily every day of treatment.

### 3 - Preventive anti-inflammatory effects of Limonene on colon inflammation

The 30 rats are weighed, marked and shared in 5 groups of 6 (n = 6) :
- "control" group = no colon inflammation and daily oral treatment with maize oil ;
- "TNBS control" group = induced colon inflammation and daily oral treatment with maize oil ;
- "TNBS + Limonene 10" group = induced colon inflammation and daily oral treatment with Limonene at 10 mg/kg ;
- "TNBS + Limonene 100" group = induced colon inflammation and daily oral treatment with Limonene at 100 mg/kg ;
- "TNBS + lbuprofen" group = induced colon inflammation and daily oral treatment with lbuprofen at 50 mg/kg.

### 3.1- Colon inflammation induction

Colon inflammation is induced by a unique rectal administration of a 2,4,6-trinitrobenzen-sulfonic acid solution (TNBS, Fluka, France). TNBS is dissolved in 40° alcohol at a concentration of 50 mg/ml. Animals are anaesthetised intraperitoneally by 2 mg/kg of Calmivet (Vetoquinol, Lure, France) and 50 mg/kg of Ketamine (Ketamine 1000, Virbac, Carros, France). 0.4 ml of the TNBS solution are administered rectally and the rats are maintained during at least 30 minutes to avoid the releasing of the TNBS.

### 3.2 - Products administration (Table 1)

Limonene is administered daily and orally at 10 and 100 mg/kg, during 3 days before and 5 days after the colon inflammation induction. Ibuprofen, used as control, is administered daily and orally at 50 mg/kg, during 3 days before and 5 days after the colon inflammation induction.

**Table 1 : summary of the treatment conditions**

| **Groups** | **Rats** | C**olon inflammation induction** | | **Treatment days** |
|---|---|---|---|---|
| | | **Product** | **Induction day** | |
| **Control** | 6 | Maize oil | J₁ | J₂ to J₅ |
| **TNBS control** | 6 | TNBS | J₁ | |
| **TNBS 10Limonene** | 6 | TNBS | J₁ | |
| **TNBS + Limonene 100** | 6 | TNBS | J₁ | |
| **TNBS + Ibuprofen** | 6 | TNBS | J₁ | |

### 4 - Statistics

Kruskal-Wallis test followed possibly by the Mann-Whitney test to compare the different studied variables of the treated groups by comparison with the groups "Control" and "TNBS/Control".Significativity threshold is settled at P < 0.05. Statistics are carried out with the Statview 5 software (SAS, Institute Inc., USA).

### RESULTS

### 1. - Animal weight progress

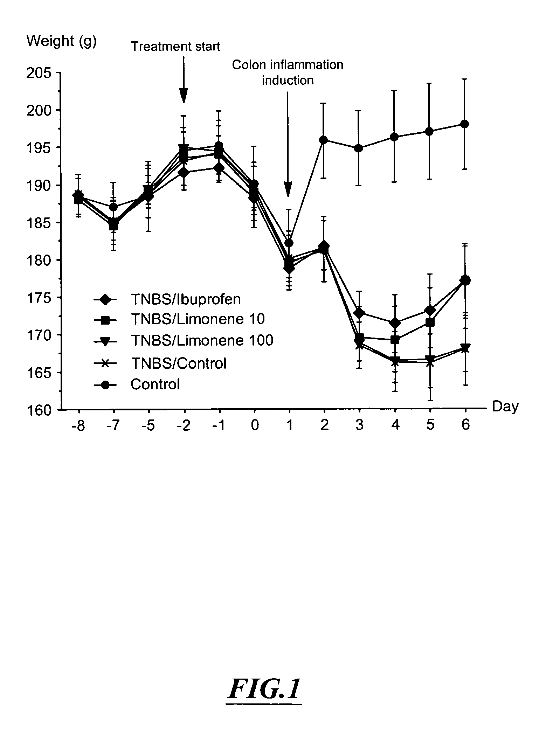

Figure 1 shows the average weight progress of the animals of the 5 groups during the assay.

Kruskal-Wallis Test (H_{(ddl=4)} = 0.381 ; *P* = 0.984) does not show significant heterogeneity among the average weights of the rats of the 5 groups of treatments before the treatment at J-2.

At J1, just before the rectally TNBS administration and after a 48 hours fasting period (J-1 toJ1), Kruskal-Wallis test (H_{(ddl=4)} = 0.308 ; P = 0.989) does not show significant heterogeneity among the average weights of the rats of the 5 groups of treatments.

At J6, Kruskal-Wallis test (H_{(ddl=4)} = 14.415 ; *P* = 0.0061) shows a significant heterogeneity among the average weights of the rats of the 5 groups of treatments. U test of Mann-Whitney shows that the rats average weights of the groups "TNBS/control", "TNBS/Limonene 10", "TNBS/Limonene 100" and "TNBS/Ibuprofen" are significantly lower than those of the rats of the group "Control". The rats average weights of the groups "TNBS/Limonene 10", "TNBS/Limonene 100" and "TNBS/Ibuprofen" are not significantly different than those of the rats of the group "TNBS/Control". The rats average weights of the groups "TNBS/Limonene 10", "TNBS/Limonene 100" and "TNBS/Ibuprofen" are not significantly.

### 2 - Colon macroscopic scores

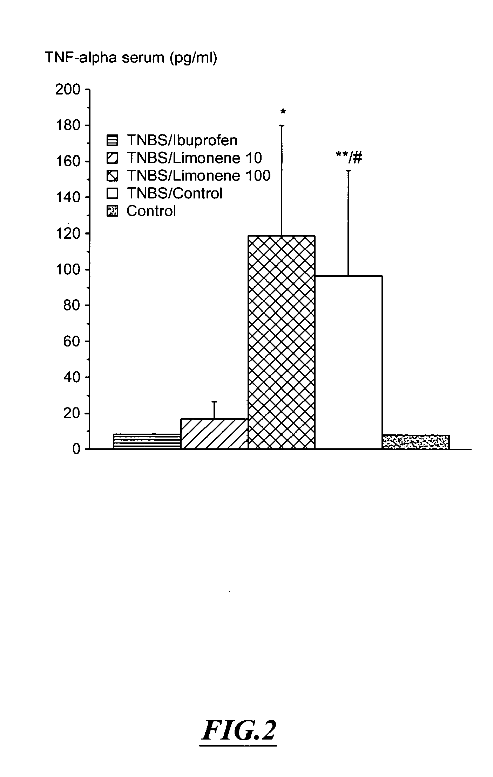

Figure 2 and Table 2 show the average macroscopic scores of the colons of the animals of the 5 groups of treatment removed 6 days after the colon inflammation induction. Kruskal-Wallis test (H_{(ddl=4)} = 21.925 ; *P* = 0.0002) shows a significant heterogeneity among the average macroscopic scores of the colons of the rats of the 5 groups of treatment. U test of Mann-Whitney shows that the average macroscopic scores of the colons of the rats of the groups "TNBS/Control", "TNBS/Limonene 10", "TNBS/Limonene 100" et "TNBS/Ibuprofen" are significantly higher than those of the rats of the group "Control". The average macroscopic scores of the colons of the rats of the groups "TNBS/Limonene 10" and "TNBS/Ibuprofen" are significantly lower than those of the rats of the groups "TNBS/Control" and "TNBS/Limonene 100" (z = 2.03 ; P = 0.0427 and z = 2.41 ; P = 0.0159, respectively). The average macroscopic scores of the colons of the rats of the groups "TNBS/Limonene 10" and "TNBS/Ibuprofen" are not significantly different.

**Table 2**

| **Treatment** | **Control** | **TNBS/Control** | **TNBS/Limonene 10** | **TNBS/Limonene 100** | **TNBS/ Ibuprofen** |
|---|---|---|---|---|---|
| | (n = 6) | (n = 6) | (n = 6) | (n = 6) | (n = 6) |
| **Mean** | 0.0 | 10.7 | 6.5 | 10.7 | 4.7 |
| **±** | ± | ± | ± | ± | ± |
| **ESM** | 0.0 | 2.0 | 1.3 | 2.7 | 2.7 |
| **Kruskal-Wallis Test** | | | | | |
| H_{(ddl=4)} = 21.925 | | z = 3.08 | z = 3.09 | z = 3.08 | z = 3.08 |
| ; *P* = 0.0002 | | | | | |
| **U Test of Mann- Whitney** | | *P* = 0.0021 | *P* = 0.0020 | *P* = 0.0021 | *P* = 0.0021 |
| (*vs.* Control) | | | | | |
| **U Test Mann-Whitney** (*vs*. TNBS/Control) | | | z = 2.50 | z = 0.33 | z = 2.59 |
| | | | **P** = 0.0124 | N.S. | *P* = 0.0097 |

### 3 - Colon length

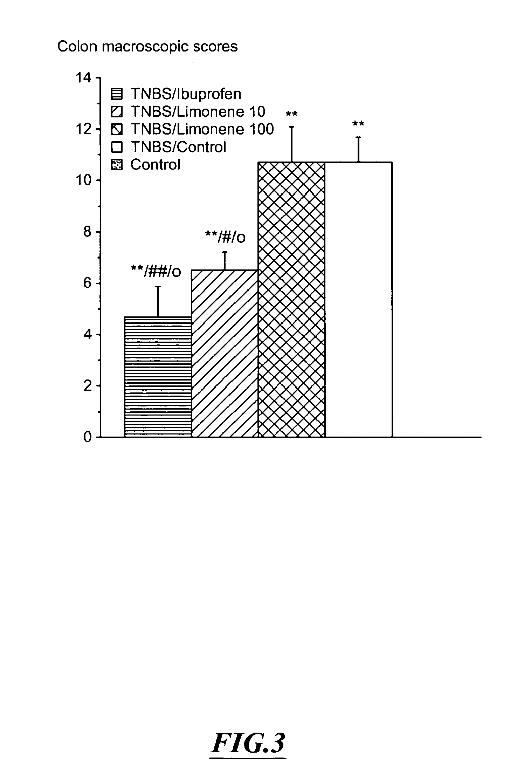

Figure 3 shows the average lengths of the colons of the animals of the 5 groups of treatment removed 6 days after the colon inflammation induction.

Kruskal-Wallis test (H_{(ddl=4)} = 15.827 ; *P* = 0.0033) shows a significant heterogeneity among the average lengths of the colons of the rats of the 5 groups of treatment. U test of Mann-Whitney shows that the average lengths of the colons of the rats of the groups "TNBS/Control", "TNBS/Limonene 10", "TNBS/Limonene 100" and "TNBS/Ibuprofen" are significantly higher than those of the rats of group "Control". The average lengths of the colons of the rats of the groups "TNBS/Limonene 10", "TNBS/Limonene 100" and "TNBS/Ibuprofen" are significantly lower than those of the rats of group "TNBS/Control". The average lengths of the colons of the rats of the groups "TNBS/Limonene 10", "TNBS/Limonene 100" and "TNBS/Ibuprofen" are not significantly different.

### 4 - Histopathological analysis and microscopic scores of the colons

Histopathological analysis of the colons of the animals of the 5 groups of treatment removed 6 days after the colon inflammation induction are carried out on 9 cut layouts for one colon.

Colons of the group "Control" show a normal colic wall, without oedema, inflammatory infiltrate, necrosis, epithelial atrophy nor dysplasy, corresponding to a microscopic score of 0.

Colons of the group "TNBS/Control" show in general a colic wall extremely reworked with acute suppurated necrotic and multifocal inflammatory lesions corresponding to a microscopic score of 4.

Colons of the group "TNBS/Limonene 10" show in general a colic wall reworked with mild multifocal lesions of ulcerous non specific colitis corresponding to a microscopic score of 2.

Colons of the group "TNBS/Limonene 100" show in general a colic wall extremely reworked with acute suppurated necrotic and multifocal inflammatory lesions corresponding to a microscopic score of 4.

Colons of the group "TNBS/Ibuprofen" show for one half a colic wall with a mild bifocal lesion of ulcerous non specific colitis corresponding to a microscopic score of 1 and for the other half a colic wall extremely reworked with acute suppurated necrotic and multifocal inflammatory lesions corresponding to a microscopic score of 4.

### Example 2 : preventive anti-inflammatory effects of limonene and perillyl alcohol in a mouse chronic skin inflammation model

### MATERIAL AND METHODS

### 1 - Animals

24 female mice hairless Skh-1 with an average weight of 20-25 g are used.

The 24 mice are marked and shared in 4 groups of 6 (n = 6) :
- "control" group = no induced skin inflammation and daily topical treatment during 10 days with carrier;
- "TPA+control" group = induced skin inflammation and daily topical treatment during 10 days with carrier ;
- "TPA+Limonene" group = induced skin inflammation and daily topical treatment during 10 days with limonene 10mg/kg/day;
- "TPA+Perillyl alcohol" group = induced skin inflammation and daily topical treatment during 10 days with perillyl alcohol 10 mg/kg/day;

### 2 - Induction of skin inflammation

Phorbol 12-myristate-13-acetate (TPA, MW = 616,83, purity = at least 98%) has been stored before use according to the manufacturer's instructions (SIGMA-ALDRICH, Saint-Quentin Fallavier, France). 100 µl of a TPA solution are applied daily on the dorsal surfaces of mice during seven days (from J₄ to J₁₀) at a concentration of 0.2 mg/ml.

### 3 -Assayed products and treatment conditions

Limonene ((R)-(+)-Limonene, MW = 136.23, purity = 97%) from SIGMA-ALDRICH (Saint-Quentin Fallavier, France) has been used and stored according to SIGMA-FLUKA instructions.

Perillyl alcohol ((S)-4-Isopropenyl-1-cyclohexenylmethanol ; (S)-p-Mentha-1,8-dien-7-ol, MW = 152,23, purity = 98%) from SIGMA-ALDRICH (Saint-Quentin Fallavier, France) has been used and stored according to SIGMA-FLUKA instructions.

Limonene and Perillyl alcohol have been prepared in maize oil extemporarily every day of treatment.

The daily topical treatment begun 3 days before the induction of skin inflammation (=J₁) and was continued 7 days after the induction of skin inflammation (=J₁₀).

From J₄ to J₁₀, the assayed products are applied 30 minutes before the application of TPA on the same skin area.

### 4 - Serum sampling

The mice are anaesthetized by intraperitoneal injection of a mixture of Ketamine (Ketamine 1000, Virbac, Carros, France) and Xylazine (Rompun 2%, Bayer Healthcare, Kiel, Allemagne).(2/3-1/3 vol/vol) at a dosis of 8 ml/kg. Blood (1 ml) is punctured intracardiacally after the last treatment. Blood samples are stored at +4°C during 20 to 30 minutes then centrifuged at 1500g for 15 minutes. Serums are removed, freezed at -20°C and stored at -80°C until pro-inflammatory cytokines dosage.

### 5 - Skin sampling

Dorsal skin samples are removed, fixed and stored in formaldehyde (Roti^{®}-Histofix 4%, Carl Roth, Karlsruhe, Allemagne) until histopathological analysis.

The animals are killed by an excessive dose of anaesthetics after the blood and skin samplings.

### 6 - Pro-inflammatory cytokines dosage

The levels of pro-inflammatory cytokines IL-1β, IL-6 et TNF-α are measured simultaneously in duplicate by the Mouse 3-plex A panel Bio-Rad kit (Ref. 171-F11080, Marnes-la-Coquette, France) in the serum samples after thawing.

### 7 - Statistics

Kruskal-Wallis test followed possibly by the Mann-Whitney test to compare the different studied variables of the treated groups by comparison with the groups "Control" and "TPA+Control". Significativity threshold is settled at P < 0.05. Statistics are carried out with the Statview 5 software (SAS, Institute Inc., USA).

### RESULTS

### 1-Animal weight progress

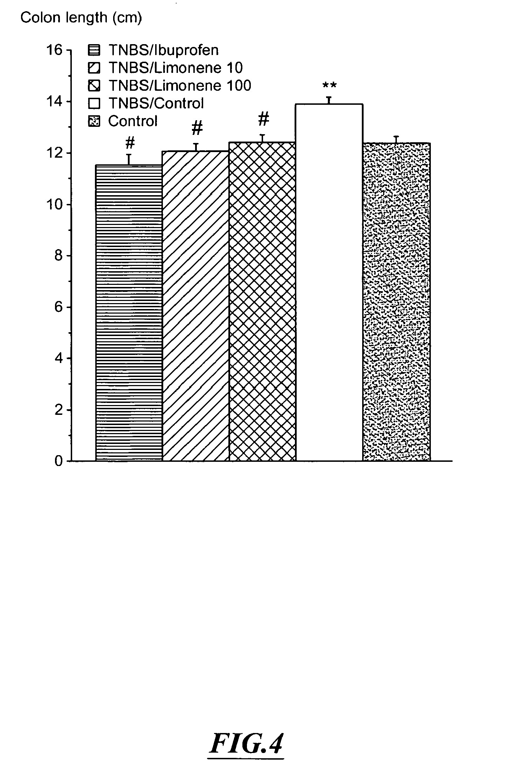

Figure 4 shows the average weight progress of the mice of the 4 groups during the assay. Between J₋₁ and J₁₀, the average weight of the mice did not show a significant difference between the different groups but a tendency to heterogeneity (H_{(ddl=3)}= 6.73, p= 0.081).

### 2- Macroscopic scores of skin inflammation

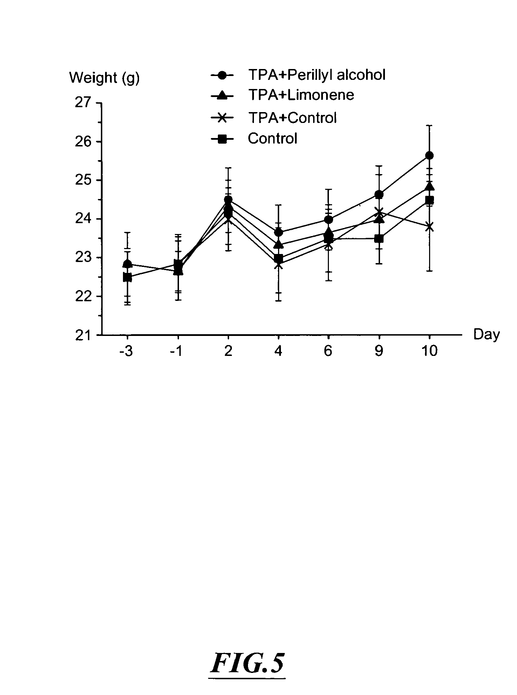

Figure 5 shows the average macroscopic scores of skin inflammation.

The average macroscopic scores of skin inflammation are significantly different between the different groups (H_{(ddl=3)}= 19.83, p = 0.0002).

The average macroscopic scores of skin inflammation of the groups TPA+control, TPA+Limonene and TPA+Perillyl alcohol are significantly higher than the one of the control group (z = 3.00, p = 0.0027 ; z = 3.11, p = 0.0019 ; z = 3.14, p = 0.0017, respectively).

The average macroscopic scores of skin inflammation of the groups TPA+Limonene and TPA+Perillyl alcohol are significantly lower than the one of the group TPA+control (z = 2.62, p = 0.0089 ; z = 2.82, p = 0.0049, respectively). The average macroscopic score of skin inflammation of the group TPA+Perillyl alcohol is significantly lower than the one of the group TPA+Limonene (z = 2.07, p = 0.039).

### 3- Histopathological analysis of the skin samples

The histopathological analysis of the skin samples removed from the mice of the different treatment groups show the following results :
- control group : skins are standard,
- TPA+control group : skins are clearly inflammatory ; show non-specific inflammatory skin lesions with epidermis ulceration and with an inflammatory infiltrate often marked,
- TPA+Limonene group : skins are inflammatory; show non-specific inflammatory skin lesions without epidermis ulceration and with an inflammatory infiltrate more or less marked,
- TPA+Perillyl alcohol group : skins are little inflammatory; show non-specific inflammatory skin lesions without epidermis ulceration and with an inflammatory rarely marked.

### 4- Microscopic scores of skin inflammation

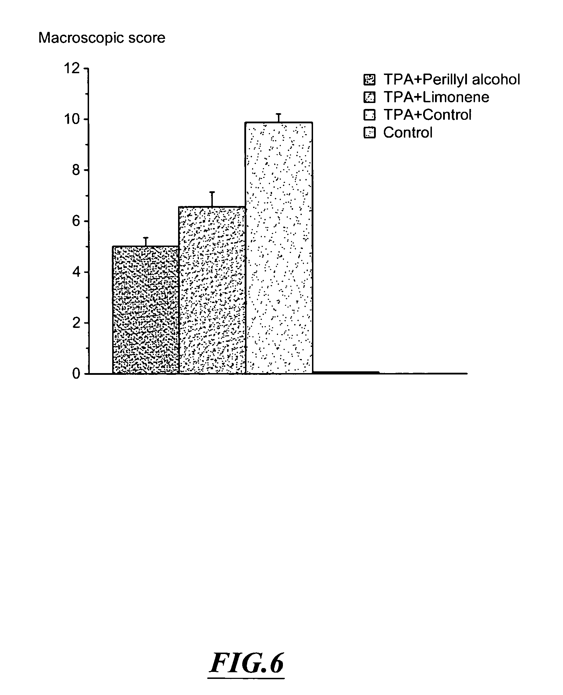

Figure 6 shows the average microscopic scores of skin inflammation of the different treatment groups.

The average microscopic scores of skin inflammation are significantly different between the different groups (H_{(ddl=3)}= 17.49, p = 0.0006). The average microscopic scores of skin inflammation of the groups TPA+control, TPA+Limonene and TPA+Perillyl alcohol are significantly higher than the one of the control group (z = 2.91, p = 0.0036 in every case). The average microscopic scores of skin inflammation of the groups TPA+Limonene and TPA+Perillyl alcohol are significantly lower than the one of the group TPA+control (z = 2.10, p = 0.036 ; z = 2.49, p = 0.013, respectively). The average microscopic scores of skin inflammation of the groups TPA+Perillyl alcohol and TPA+Limonene are not significantly different (z = 0.97, p = 0.33).

### 5- Pro-inflammatory cytokines dosages

### 5.1. IL-1β

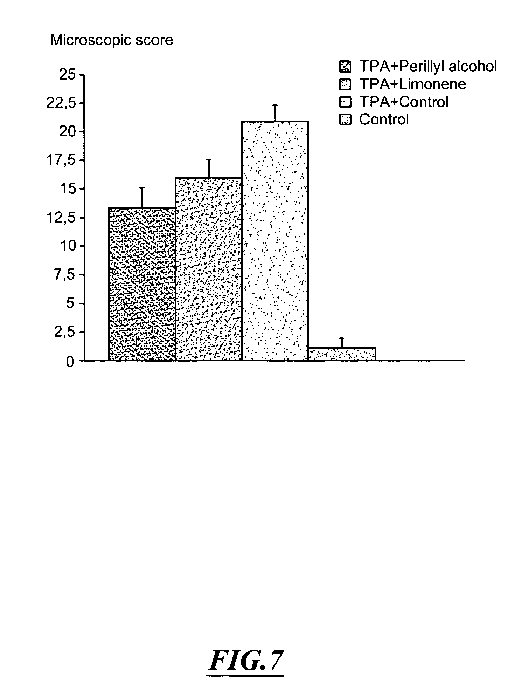

Figure 7 shows the average serum levels of IL-1β of the different treatment groups.

The average serum levels of IL-1β of the different treatment groups are significantly different (H_{(ddl=3)}= 15.62, p = 0.0014) (Table 3).

The average serum levels of IL-1β of the TPA+control, TPA+Limonene and

TPA+Perillyl alcohol groups are significantly higher than the one of the control group (z = 3.08, p = 0.0021 in every case).

The average serum level of IL-1β of the TPA+Perillyl alcohol group shows a tendency to be significantly lower than the one of the TPA+control group (z = 1.92, p = 0.055).

No significant difference has been showed between the average serum levels of IL-1β of the TPA+Limonene and TPA+Perillyl alcohol groups.

**Table 3**

| **Average serum levels of IL-1β (µg/ml) of the mice of the different treatment groups (Mean ± ESM)** | | | | |
|---|---|---|---|---|
| **Group** | **Control** | **TPA + Control** | **TPA + Limonene** | **TPA alcohol** |
| **Serum levels of IL-1β** | 0.0 ± 0.0 | 9.3 ± 2.9 | 6.5 ± 1.8 | 5.3 ± 1.7 |

5.2. IL-6

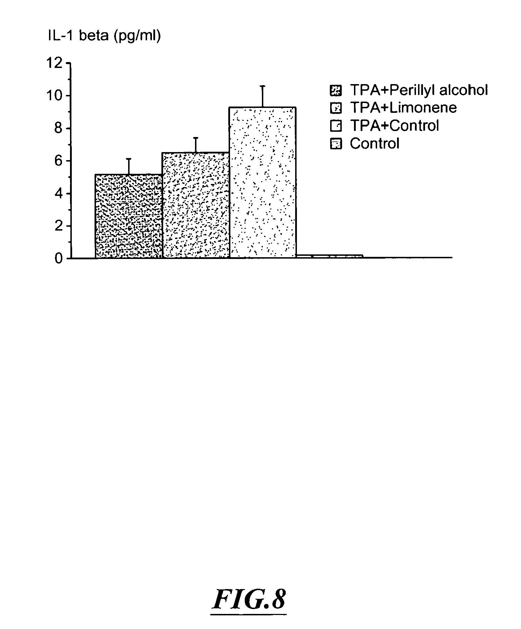

Figure 8 shows the average serum levels of IL-6 of the different treatment groups.

The average serum levels of IL-6 of the different treatment groups are significantly different (H_{(ddl=3)}= 16.42, p = 0.0009) (Table 4).

The average serum levels of IL-6 of the TPA+control, TPA+Limonene and

TPA+Perillyl alcohol groups are significantly higher than the one of the control group (z = 3.08, p = 0.0021 in every case).

The average serum level of IL-6 of the TPA+Perillyl alcohol group is significantly lower than the one of the TPA+control group (z = 2.08, p = 0.037).

The average serum level of IL-6 of the TPA+Limonene group shows a tendency to be significantly lower than the one of the TPA+control group (z = 1.76, p = 0.078). No significant difference has been showed between the average serum levels of IL-6 of the TPA+Limonene and TPA+Perillyl alcohol groups.

**Table 4**

| **Average serum levels of IL-6 (pg/ml) of the different treatment groups (Mean ± ESM)** | | | | |
|---|---|---|---|---|
| **Group** | **Control** | **TPA + Control** | **TPA + Limonene** | **TPA alcohol** |
| **Serum levels of IL-6** | 0.0 ± 0.0 | 22.0 ± 5.5 | 15.3 ± 3.0 | 13.0 ± 3.8 |

5.8. TNF-α

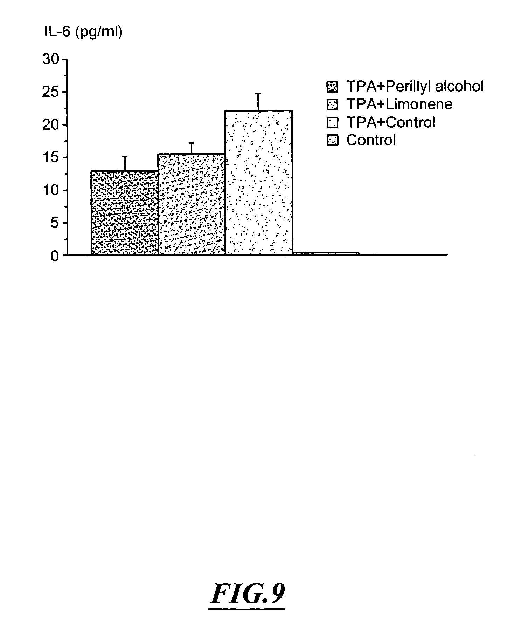

Figure 9 shows the average serum levels of TNF-α of the different treatment groups.

The average serum levels of TNF-α of the different treatment groups are significantly different (H_{(ddl=3)}= 17.08, p = 0.0007) (Table 5).

The average serum levels of TNF-α of the TPA+control, TPA+Limonene and

TPA+Perillyl alcohol groups are significantly higher than the one of the control group (z = 3.08, p = 0.0021 in every case).

The average serum level of TNF-α of the TPA+Perillyl alcohol group is significantly lower than the one of the TPA+control group (z = 2.24, p = 0.025).

The average serum level of TNF-α of the TPA+Limonene group shows a tendency to be significantly lower than the one of the TPA+control group (z = 1.76, p = 0.078).

No significant difference has been showed between the average serum levels of TNF-α of the TPA+Limonene and TPA+Perillyl alcohol groups.

**Table 5**

| **Average serum levels of TNF-α (pg/ml) of the different treatment groups (Mean ± ESM)** | | | | |
|---|---|---|---|---|
| **Group** | **Control** | **TPA + Control** | **TPA + Limonene** | **TPA + Perillyl alcohol** |
| **Serum levels of TNF-α** | 0.0±0.0 | 131.9±18.9 | 103.2±18.0 | 80.6±24.7 |

### 6- Conclusion

Limonene and Perillyl alcohol, topically administered in preventive treatment during 3 days at 10 mg/kg/day before induction of skin inflammation, show a significant effect on skin inflammation by reducing the inflammation degree (macroscopic and microscopic scores) and by reducing the pro-inflammatory cytokines secretion (IL-1β, IL-6 et TNF-α).

### Example 3 : effects of limonene and perillyl alcohol on healing

### MATERIAL AND METHODS

### 1 - Animals

6 female mice hairless Skh-1 with an average weight of 20-25 g are used.

The 6 mice are marked and shared in 3 groups of 2 :
- "control" group = scarification on each flank and daily topical treatment during 8 days with carrier;
- "Limonene" group = scarification on each flank and daily topical treatment during 8 days with limonene 10mg/kg/day;
- " Perillyl alcohol" group = scarification on each flank and daily topical treatment during 8 days with perillyl alcohol 10 mg/kg/day;

### 2 - Scarification

Each mouse is submitted to a scarification of the skin on each flank.

### 3 -Assayed products and treatment conditions

Limonene ((R)-(+)-Limonene, MW = 136.23, purity = 97%) from SIGMA-ALDRICH (Saint-Quentin Fallavier, France) has been used and stored according to SIGMA-FLUKA instructions.

Perillyl alcohol ((S)-4-Isopropenyl-1-cyclohexenylmethanol ; (S)-p-Mentha-1,8-dien-7-ol, MW = 152,23, purity = 98%) from SIGMA-ALDRICH (Saint-Quentin Fallavier, France) has been used and stored according to SIGMA-FLUKA instructions.

Limonene and Perillyl alcohol have been prepared in maize oil extemporarily every day of treatment.

The daily topical treatment begun 1 hour before the scarification and was continued 8 days after the scarification.

### RESULTS

Daily observations and photographs were performed in order to follow the healing process.

The wound healing process gave better results with daily topical application of a maize oil solution of perillyl alcohol in comparison with limonene and much better results in comparison with corn oil alone.

Less marks of healing were also observed at the sites of scarification, at the skin surface and under the skin surface.

### REFERENCES

- Crowell PL et al., "Human metabolism of the experimental cancer therapeutic agent d-limonene", Cancer Chemother Pharmacol. 1994;35(1):31-7;
- Dong Y-L, Fleming RYD, Yan TZ, Herndon DN, Waymack JP. Effect of ibuprofen on the inflammatory response to surgical wounds. Journal of Trauma. 1993; 35(3):340-343;
- Dvivedi S, Tiwari SM, Sharma A. Effect of ibuprofen and diclofenac sodium on experimental wound healing. Indian Journal of Experimental Biology. 1997; 35(11):1243-1245;
- Hardcastle IR et al., "Inhibition of protein prenylation by metabolites of limonene", Biochem Pharmacol. 1999 Apr 1;57(7):801-9;
- Kulick MI, Smith S, Hadler K. Oral ibuprofen : evaluation of its effect on peritendinous adhesions and the breaking strength of a tenorrhaphy. Journal of Hand Surgery. 1986; 11(1):110-120;
- Kumar A, Rao M, Kulkarni DR. Zinc incorporation reverses suppressant effect of ibuprofen on wound healing. Indian Journal of Experimental Biology. 1988;26(6):483-485.
- Lee KH. Studies on the mechanism of action of salicylate. II. Retardation of wound healing by aspirin. Journal of Pharmaceutical Sciences. 1968; 57(6):1042-1043;
- Rao CM, Kumar A, Kulkarni DR., Effects of enfenamic acid and its zinc salt on wound-heaking. Indian Journal of Physiology and Pharmacology. 1988; 32(1):61 - 66;
- Stanley P.L., Steiner S;, Havens M., Tramposch K.M., Skin Pharmacol., 4, 262-271, 1991;
- Vigushin DM et al., « Phase I and pharmacokinetic study of D-limonene in patients with advanced cancer. Cancer Research Campaign Phase I/II Clinical Trials Committee", Cancer Chemother Pharmacol. 1998;42(2):111-7;

## Claims

1. Use of a monoterpene to increase biological tissue repair of *in vitro* or ex *vivo* cultured tissues.

2. Cosmetic use of a monoterpene to increase skin or mucosa tissue repair in an individual.

3. The use according to claim 2, to increase a skin defect repair.

4. The use according to claim 3, said skin defect being selected from the group consisting of acne pimples, cellulitis, scars, sun and UV induced premature skin senescence, unesthetic skin defaults due to the aging process including scars and stains, wrinkles and squamous features, stretch marks, aging marks, sun and UV induced premature skin senescence, rosacea and unesthetisms of post-traumatic relapses.

5. The use according to any of claims 1 to 4, to increase biological tissue regeneration.

6. Use of a monoterpene for the manufacture of a pharmaceutical composition intended to prevent or treat a biological tissue injury in an individual.

7. The use according to claim 6, said tissue injury being selected in the group consisting of burns, cuts, relapses of extreme weather conditions including excessive cold or heat, burning relapses and wounds.

8. Use of a monoterpene for the manufacture of a pharmaceutical composition intended to prevent or treat a skin disease in an individual.

9. The use according to claim 8, said tissue disease being a skin disease selected from the group consisting of atopic dermatitis, seborrheic keratitis, epidermolysis bullosa acquisita, psoriasis, skin alterations in lupus erythematosus, dermatomyositis, scleroderma, chronic acne, chronic cellulites, pruritus and abnormal or defective scar formation in diabetes.

10. The use according to any of claims 6 to 9, wherein said pharmaceutical composition is for topical application.

11. The use according to any of claims 2 to 10, said individual being a human.

12. The use according to any of the preceding claims, said monoterpene being selected from the group consisting of limonene, geraniol, geranyl acetate, isomenthone, perillyl alcohol, perillic acid, dihydroperillic acid, the respective methyl esters of these acids, carvone, citral, and menthol.

13. The use according to claim 12, said monoterpene being limonene or perillic acid or perillyl alcohol or a mixture thereof.
